# EUROPEAN PATENT APPLICATION

(11) **EP 2 319 498 A1**
(43) Date of publication of application: **11.05.2011**
(21) Application number: 10184903.2
(22) Date of filing: 13.10.2005
(51) Int. Cl.: A61K 9/16, A61K 9/20, A61K 9/48, A61K 31/495, A61K 31/4045

(54) **Taste-masked multiparticulate pharmaceutical composition comprising a drug-containing core particle and a solvent-coacervated membrane**

(30) Priority: 12.11.2004 US 627525 P; 26.08.2005 US 213266
(62) Divisional of application: 05851221.1
(71) Applicant: Eurand Pharmaceuticals Ltd, Southern Cross Road Bray, Co. Wicklow (IE)
(72) Inventor: Lai, Jin-wang, Springboro, Ohio 45066 (US); Qian, Ken, Manchester, CT 06042 (US); Venkatesh, Gopi, Vandalia, Ohio 45377 (US)
(74) Representative: Gowshall, Jonathan Vallance

(57) **Abstract**

There is provided a method for preparing an orally disintegrating tablet (ODT) composition comprising microparticles of one or more taste-masked active pharmaceutical ingredients, rapidly-dispersing microgranules, and other optional, pharmaceutically acceptable excipients wherein the ODT disintegrates rapidly with saliva in the buccal cavity forming a smooth, easy-to-swallow suspension. Furthermore, the microparticles (crystals, granules, beads or pellets containing one or more actives) with a taste-masking membrane applied by a modified solvent coacervation process comprising a water-insoluble polymer and at least one gastrosoluble inorganic or organic pore-former, exhibit a pleasant taste when placed in the oral cavity and provide rapid, substantially-complete release of the dose on entry into the stomach.

## Description

### CROSS REFERENCE TO RELATED APPLICATIONS

The present application claims the benefit of U.S. Provisional Application No. 60/627,525 filed November 12, 2004.

### TECHNICAL FIELD

This invention relates to an orally disintegrating tablet (ODT) composition comprising taste-masked microparticles of one or more active pharmaceutical ingredient(s) suitable for oral administration for the treatment of diseases and rapidly-dispersing microgranules comprising a disintegrant and a sugar alcohol or a saccharide, or a mixture thereof, each sugar alcohol or saccharide having an average particle diameter of not more than about 30 µm. The multi-particulate ODT composition contains rapidly-dispersing microgranules and drug-containing core particles (crystals or granules, beads or pellets of one or more active pharmaceutical ingredients) microencapsulated by coacervation (phase separation) with a taste-masking membrane comprising a water-insoluble polymer in combination with one or more pore-formers such as inorganic or organic salts which are practically insoluble in water and saliva, but soluble in an acidic buffer. The ODT composition rapidly disintegrates on contact with saliva when placed in the oral cavity forming a smooth, easy-to-swallow suspension containing coated particles exhibiting acceptable taste-masking and provides rapid, substantially-complete release of the dose on entry into the stomach, thereby enhancing the probability of achieving bioequivalence to an immediate-release (IR) product. The invention additionally provides a method of manufacturing orally disintegrating tablets comprising rapidly-dispersing microgranules and acceptably taste-masked microparticles (crystals, pellets, granules, or beads containing the drug) with an average particle size of not more than about 400 µm, more particularly not more than about 300 µm, to provide a smooth mouthfeel leaving no aftertaste (non-gritty or non-chalky taste) after swallowing the suspension.

### BACKGROUND OF THE INVENTION

There are two types of most widely used dosage forms for medication by oral administration: tablets and capsules. However, such dosage forms have several disadvantages. For example, it is estimated that 50% of the population have problems swallowing tablets (see Seager in Journal of Pharmacol. and Pharm. 50, pages 375-382, 1998); especially it is hard for aged persons to swallow tablets or capsules or to medicate children who are unable or unwilling to swallow tablets or capsules. This leads to poor, even non-compliance with the treatment and thus has a negative impact on the efficacy of the treatment. The bitter taste of many actives precludes the medication from being easily sprinkled onto food such as applesauce, a commonly used method of administering medications to children. The conventional capsule or tablet dosage form is also inconvenient for the 'people on the move' who often do not have access to drinking water or fluids. Chewable tablets comprising taste-masked particles capable of being chewed without experiencing a bitter taste were introduced not too long ago, and these tablets became popular with children.

The bitter drug-containing cores incorporated into chewable tablets typically have thick coatings of mostly water-insoluble polymers such as ethylcellulose to resist fracture during tablet compression and/or during chewing and concomitant leakage of the bitter active. Consequently, substantially complete release of the drug from such chewable tablets in the gastrointestinal tract may take 2 hours or longer. More recently, orally disintegrating tablet (ODT) dosage forms have been introduced, which rapidly dissolve or disintegrate in the buccal cavity and hence can be taken without water. Such medicines are convenient for all, especially for aged persons, children or 'people on the move'.

An ideal orally disintegrating tablet formulation comprising rapidly-dispersing microgranules and drug-containing microparticles (crystals, pellets, granules, or beads containing the drug) with a taste-masking membrane (if required) should rapidly disintegrate on contact with saliva in the oral cavity forming a smooth, easy-to-swallow suspension containing taste-masked drug particles having an average particle diameter of not more than about 400 µm to provide a smooth mouthfeel leaving no aftertaste (i.e., little or minimal drug release with a non-gritty or non-chalky taste) until swallowed, and should provide rapid, substantially-complete release upon arrival in the stomach in order to be bioequivalent to the reference product.

As indicated earlier, most of the active pharmaceutical ingredients in the market are bitter to a varying degree. Typically, to eliminate/minimize drug-release in the oral cavity, the bitter drug substance was taste-masked in the prior art by providing a thick polymer-membrane around the drug particle typically by microencapsulation (coacervation by phase separation) or fluid-bed coating for preparing immediate release dosage forms (chewable tablets, sprinkles, sachets, suspensions). However, coating with water-insoluble polymers such as ethylcellulose (EC), cellulose acetate (CA), cellulose acetate phthalate, polyvinyl acetate, Eudragit® RS, RL, L, S and NE30D polymers, results in slower dissolution profiles and not-too-infrequently results in imparting sustained-release properties.

Several marketed products, which are typically conventional or effervescent based immediate-release dosage forms, exhibit a rapid-onset of action with a Tₘₐₓ of about an hour or less. An undesirable consequence of taste-masking using a water-insoluble polymer alone or in combination with a water-soluble polymer is in general the slower release of the drug in the gastrointestinal tract. Eudragit EPO or E100, a copolymer consisting of dimethylaminoethyl methacrylate and neutral methacrylic acid esters with a weight-average molecular weight of 150,000 and a pKₐ of 6.3, is soluble in gastric fluid below pH 5 while it swells and/or is permeable in water and buffer solutions above pH 5.0. The saliva is typically in the pH range of 6.7 to 7.4. Hence, it is likely that one achieves effective taste-masking in the oral cavity, although for a very limited time duration, if the drug core is coated with Eudragit E100/EPO alone or in combination with a water-soluble agent.

The method of producing taste-masked microparticles (mean particle size of about 100-400 *µ*m) in accordance with the present invention comprising one or more bitter active pharmaceutical ingredient(s) includes microencapsulation (solvent coacervation by phase separation) of drug-containing core particles (crystals, microgranules, drug-layered or extruded/spheronized-beads) with a mixture of a water-insoluble polymer such as ethylcellulose and one or more gastrosoluble pore-former(s) such as inorganic or organic salts, at a ratio of about 50/50 to 95/5 for a weight gain of not less than about 5% and not more than about 50% by weight, based on total weight of the coated particle.

This improved solvent coacervation process includes adding/suspending a micronized pore-forming agent to the coacervation tank at an elevated temperature, typically from about 50°C-65°C, more specifically about 58°C during the forming-hardening phase of the microcapsule-membrane, resulting in the pore-former being distributed throughout the taste-masking membrane. In accordance with particular embodiments, the pore-former may be distributed substantially uniformly throughout the taste-masking membrane. The gastrosoluble pore-forming agent is insoluble in both water and saliva (pH range: 6.7 to 7.4), but soluble in a gastric fluid. The membrane so formed provides adequate taste-masking of the drug particles in the oral cavity as the pore-former is insoluble at salivary pH. Once the taste-masked particles reach the acidic environment of the stomach, however, the pore-former rapidly dissolves, thereby releasing the microencapsulated drug in the stomach. Alternatively, the modified coacervation comprises initially charging the drug, the membrane material and the pore-former into the coacervation tank and following the heating and cooling cycles to produce taste-masked microcapsules with similar properties.

Furthermore, the microcapsules prepared in accordance with the present invention can be produced to exhibit the specified criteria (viz., desired particle size distribution and little or minimal release of the bitter active in the mouth (hence no aftertaste), and rapid-release of the dose from the taste-masked microparticles upon entry into the stomach), to be suitable for incorporation into orally disintegrating tablets.

The taste-masking effectiveness is measured by % of the dose released in a simulated saliva fluid at a pH of 6.7-7.4. The smaller the % release, the more effective the taste-masking. A pharmaceutical composition with not more than 10% of the dose released in about 3 minutes in a simulated saliva fluid (the longest anticipated residence time for taste-masked microparticles in the mouth) is considered acceptably taste-masked. On the other hand, the drug release on oral administration is evaluated by measuring % of the dose released in an acidic pH of about 1.2. The faster the release of the drug from the taste-masked microparticles in the stomach, the higher the probability of being bioequivalent to the reference product. A release of not less than about 75% of the dose in about 30 minutes in the acidic buffer is considered acceptable for achieving bioequivalence to the reference product.

### SUMMARY OF THE INVENTION

The present invention provides pharmaceutical compositions and methods for making taste-masked microparticles and orally disintegrating tablets, which provide effective taste-masking, smooth mouthfeel (little or no aftertaste) and rapid/complete release upon reaching the stomach.

The multi-particulate compositions comprise taste-masked core particles (crystals or granules, beads or pellets comprising one or more bitter-tasting active pharmaceutical ingredient(s)) produced by solvent coacervation with a mixture of a water-insoluble polymer (e.g., ethylcellulose) and a gastrosoluble inorganic or organic pore-former (e.g., calcium carbonate). The taste-masked composition prepared in accordance with the present invention rapidly releases the drug, i.e., not less than 75% of the dose released in 30 minutes, when tested for dissolution using United States Pharmacopoeia Apparatus 1 (baskets @ 100 rpm) or Apparatus 2 (paddles @ 50 rpm) in 900 mL of 0.1N HCl. Another embodiment of the invention relates to a pharmaceutical composition in the form of an orally disintegrating tablet comprising (i) rapidly-dispersing microgranules comprising (a) a disintegrant and (b) a sugar alcohol, saccharide or mixture thereof having an average particle size is not more than about 30 µm, (ii) microparticles of one or more bitter-tasting active pharmaceutical ingredient(s) taste-masked by solvent coacervation with a polymer membrane comprising a blend of (a) a water-insoluble polymer and (b) a gastrosoluble inorganic/organic pore-former, and (iii) optionally other pharmaceutically acceptable excipients. These orally disintegrating tablets have the properties of disintegrating on contact with saliva in the buccal cavity in about 60 seconds forming a smooth easy-to-swallow suspension with no aftertaste (good creamy mouthfeel) and rapidly releasing the dose on entry into the stomach, thus enhancing the probability of being bioequivalent to the reference product.

A taste-masked multiparticulate pharmaceutical composition comprising:
(a) a drug-containing core particle (crystal, granule, pellet, bead and the like); and
(b) a taste-masking membrane on said drug-containing core particle comprising a combination of a water-insoluble polymer and a gastrosoluble pore-former, such as calcium carbonate or calcium saccharide, at a ratio ranging from about 95/5 to about 50/50 having a thickness of from about 5% to about 50% based on the weight of the coated particle and an average particle size of not more than about 400 µm is disclosed.

The composition typically exhibits acceptable taste-masking when the composition is placed in the oral cavity for 3 minutes, more particularly for 2 minutes and in some cases for 60 seconds, most preferably until it is swallowed leaving little or no aftertaste (i.e., experiencing no gritty or chalky taste) and the composition provides rapid, substantially-complete release of the dose upon entry into the stomach, i.e., releases not less than about 75% of the dose in 30 min when tested for dissolution using United States Pharmacopoeia Apparatus 1 (Baskets @ 100 rpm in 900 mL of pH 1.2 buffer).

A taste-masked multiparticulate pharmaceutical composition in the ODT (orally disintegrating tablet) form, which disintegrates on contact with saliva in the buccal cavity in about 60 seconds forming a smooth easy-to-swallow suspension (no gritty or chalky aftertaste) is also disclosed. The ODT may comprise the drug-containing core particle (crystal, granule, pellet, bead and the like), with a taste-masking membrane on the drug-containing core particle. The taste-masking membrane may comprise a water-insoluble polymer and a gastrosoluble pore-former such as calcium carbonate at a ratio ranging from about 95/5 to about 50/50 having a thickness of from about 5% to about 50% based on the weight of the coated microparticle with an average particle size of not more than about 400 µm, or in some embodiments not more than about 300 µm, or in some embodiments not more than about 200 µm, comprising a sugar alcohol, a saccharide or a combination thereof, each having an average particle diameter of not more than about 30 µm, and optionally pharmaceutically acceptable excipients typically used in ODT formulations, viz., flavors, a sweetener, coloring agents, and additional disintegrants.

The ODT in accordance with one embodiment exhibits the following properties:
(1) disintegrates on contact with saliva in the oral cavity in about 60 seconds forming a smooth, easy-to-swallow suspension comprising taste-masked microparticles and
(2) taste-masked microparticles provide rapid, substantially-complete release of the dose upon entry into the stomach.

The ODT may comprise taste-masked microparticles demonstrating effective taste-masking by releasing not more than about 10% in about 3 minutes (the longest typical residence time anticipated for the ODT in the buccal cavity) when dissolution tested in a simulated saliva fluid (pH ∼7.0) while releasing not less than about 75% of the dose in about 30 minutes when dissolution tested in 0.1N HCl.

A method of manufacturing a taste-masked multi-particulate composition wherein the dosage form comprises one or more active pharmaceutical ingredient(s) in sufficient quantities to be administered orally to a patient at prescribed dosing regimen to provide therapeutic efficacy is also provided.

The taste-masked multiparticulate pharmaceutical composition may include any pharmaceutically acceptable active ingredient requiring taste-masking.

These and other embodiments, advantages and features of the present invention become clear when detailed description and examples are provided in subsequent sections.

### BRIEF DESCRIPTION OF THE FIGURES

The invention will be described in further detail with reference to the accompanying figures wherein:
Fig. 1 shows drug-release profiles for cetirizine ODT is described in of Examples 2 and 3 compared to commercially-available products;
Fig. 2 illustrates the plasma concentration profiles for the formulations tested in the study described in Example 4; and
Fig. 3 illustrates the dissolution profiles for sumatriptan succinate ODT is described in for Examples 5 and 6 compared to commercially-available products.

### DETAILED DESCRIPTION OF THE INVENTION

All documents cited are, in relevant part, incorporated herein by reference; the citation of any document is not to be construed as an admission that it is prior art with respect to the present invention.

The term 'drug', 'active' or 'active pharmaceutical ingredient' as used herein is meant to include the base, any pharmaceutically acceptable salt, stereo-isomer and mixtures thereof. The term represents any therapeutic agent indicated for oral administration. Examples of therapeutic agents include, but are not limited to, NSAID analgesic, histamine H₁-receptor antagonist, histamine, H₂-receptor antagonist, 5-HT₁ receptor agonist, 5-HT₃ receptor antagonist, antiepileptic drug, centrally acting adrenergic agonist, sleep-aid, leukotriene receptor antagonist, or a drug for the treatment of erectile dysfunction requiring taste-masking. Specific examples of the therapeutic agent used in various embodiments of this invention include one or more from the group consisting of sumatriptan, electriptan, cetirizine, zafirlukast, montelukast, famotidine, ranitidine, tiagabine, fexofenadine, tizanidine, alphrazolum, ondansetron, granisetron, zolpidem, zaleplon, sildenafil, tadalafil or the like.

Unless indicated otherwise, all percentages and ratios are calculated by weight. Unless indicated otherwise, all percentages and ratios are calculated based on the total composition.

An aqueous or a pharmaceutically acceptable solvent medium may be used for preparing drug-containing core particles for taste-masking, viz., beads by drug-layering onto inert sugar spheres in fluid-bed equipment. The type of film-forming binder that is used to bind the water-soluble drug to the inert sugar sphere is not critical but usually water-soluble, alcohol-soluble or acetone/water soluble binders are used. A binder, such as polyvinylpyrrolidone (PVP), polyethylene oxide, hydroxypropyl methylcellulose (HPMC), or hydroxypropylcellulose (HPC), may be used at concentrations of about 0.5 to 10 weight % based on the drug-layered beads. The drug substance may be present in this coating formulation in solution form or may be suspended at a solid content up to 35% by weight depending on the viscosity of the coating formulation.

Crystals of a bitter API with a desired particle size range of from about 20 *µ*m to 500 *µ*m, more particularly from about 50 *µ*m to 300 *µ*m may be taste-masked directly. Alternatively, microgranules containing milled or micronized drug may be produced by granulating in a high-shear granulator the active and a suitable filler/diluent (if required) with a polymeric binder, which imparts resilient characteristics to the dried microgranules to resist attrition due to stirring during solvent coacervation for taste-masking. The relative amounts of active and optional filler/diluent may vary considerably depending on the particular active and the dosage form. Typically, microgranules prepared in accordance with this aspect of the invention will contain from about 20% to about 90% active, and up to about 15% binder with any optional filler/diluent being present at from about 0 to 80% by weight of the microgranules.

Examples of useful polymeric binders include, but are not limited to, hydroxypropylcellulose (Klucel® LF from Aqualon), modified starch (e.g., Starch 1551 and Starch 1500, commercially available from National Starch and Colorcon, respectively), Kollidon® VA 64, poly(vinyl acetate-vinyl pyrrolidone) from BASF, and hydroxypropyl methylcellulose with a viscosity of 100 cps or more (e.g., Methocel K100LV and Metolose K400 commercially available from Dow Chemical and Shin Etsu Chemicals, respectively) alone or in combination with a widely used binder such as PVP (polyvinylpyrrolidone) or hydroxypropyl methylcellulose with a viscosity of 15 cps or less.

Examples of useful pharmaceutically acceptable fillers/diluents include, but are not limited to, mannitol, lactose, microcrystalline cellulose, potassium sulfate, calcium phosphate, modified starch and mixtures thereof.

The water-insoluble polymers suitable for taste-masking of bitter drugs by solvent coacervation include, but are not limited to, ethylcellulose, polyvinyl acetate, cellulose acetate, cellulose acetate butyrate, methacrylate copolymers available under the trade name of 'Eudragit' (type RL, RS and NE30D).

The gastrosoluble organic or inorganic pore-former is insoluble in water and saliva but is readily soluble under acidic conditions. In accordance with particular embodiments, the gastrosoluble pore formers are selected from the gastrosoluble oxides, hydroxides and salts of organic and inorganic acids. Examples of useful pore-formers include, but are not limited to, calcium carbonate, calcium phosphate, calcium saccharide, calcium succinate, calcium tartrate, ferric acetate, ferric hydroxide, ferric phosphate, magnesium carbonate, magnesium citrate, magnesium hydroxide, magnesium phosphate, and the like and mixtures thereof. The ratio of water-insoluble polymer to gastrosoluble organic or inorganic pore-former for producing taste-masked particles may typically vary from about 95/5 to about 50/50, or in some embodiments from about 85/15 to 65/35, at a thickness of from about 5% to about 50%, more particularly from about 10% to about 30% by weight of the coated bead.

The ODT compositions described herein also include rapidly-dispersing microgranules. One or more sugar alcohols and/or saccharides and a disintegrant may be granulated in a high shear granulator and dried in a fluid bed equipment to produce rapidly-dispersing microgranules. Rapidly dispersing microgranules typically will contain disintegrant and sugar alcohol and/or saccharide at a ratio varying from about 90/10 to about 95/5. Examples of useful sugar alcohols include, without limitation, mannitol, sorbitol, xylitol, maltitol and mixtures thereof. Examples of useful saccharides include, but are not limited to, lactose, sucrose, maltose and mixtures thereof. Each sugar alcohol or saccharide is characterized by an average particle size of not more than about 30 µm. A disintegrant or a so-called super-disintegrant may be selected from the group consisting of crospovidone (crosslinked PVP), sodium starch glycolate, crosslinked sodium carboxymethyl cellulose, low substituted hydroxypropylcellulose and mixtures thereof.

The ODT compositions may also include additional disintegrant separate from the rapidly dispersing microgranules. The additional disintegrant may be present in the ODT formulation at up to about 10% based on the tablet weight.

It is to be understood that while the invention has been described in conjunction with specific embodiments thereof, that the description above as well as the examples that follow are intended to illustrate and not limit the scope of the invention. Any modification within the scope of the invention will be apparent to those skilled in the art to which the invention pertains.

An exemplary method of producing taste-masked microparticles (mean particle size of about 100-400 *µ*m) comprising one or more bitter active pharmaceutical ingredient(s) includes (i) preparing drug-containing particles (crystals with a desired particle size range, microgranules, drug-layered or extruded/spheronized-beads) and (ii) membrane-coating these drug-containing particles for taste-masking. Crystals of a bitter API may be directly taste-masked by solvent coacervation if the drug substance with a desired particle size range of from about 20 *µ*m to 500 *µ*m, more particularly from about 50 *µ*m to 300 *µ*m, is available. Drug-containing particles for taste-masking may be produced by the method in accordance with other aspects of the invention. The method of producing drug-layered beads in one embodiment of the invention comprises dissolving or suspending one or more active pharmaceutical ingredient(s) in a polymeric binder solution and layering onto inert particles such as sugar spheres or Celphere (50-100 mesh or 150-300 *µ*m) using a fluid-bed coater equipped with a bottom-spray Wurster insert. Alternatively, an embodiment of the method of producing resilient drug-containing microgranules, which undergo little or minimal attrition during microencapsulation by solvent coacervation, includes granulating one or more actives and a filler or diluent (if needed) with a polymeric binder solution in a high-shear granulator. Yet another embodiment of the method of producing drug-containing beads involves granulating the active in a high-shear granulator as described above, followed by extrusion and spheronization of the wet mass using extrusion-spheronization equipment.

The method of producing taste-masked microparticles (crystals, microgranules, drug-layered or extruded/spheronized-beads) in accordance with the invention includes solvent coacervation with a mixture of a water-insoluble polymer such as ethylcellulose and a gastrosoluble inorganic or organic pore-former such as calcium carbonate or magnesium oxide at a ratio of about 50/50 to 95/05 for a weight gain of from about 5% to about 50%, more particularly from about 10% to about 30%.

One specific embodiment of the invention comprises dissolving water-insoluble ethylcellulose in cyclohexane at 80°C and suspending the drug-containing particles in the coacervation tank. Examples of such a coacervation process are disclosed in U.S. Pat. Nos. 5,252,337, 5,639,475, 6,139,865 and 6,495,160, which are incorporated herein by reference. During the temperature-programmed cooling cycle, the micronized pore-former is introduced into the tank at the temperature of about 58°C while constantly stirring to distribute uniformly in the microcapsule-membrane being at the forming-hardening phase. Upon reaching ambient temperature, the microcapsules are filtered, washed with fresh cyclohexane and dried to reduce residual solvent levels within acceptable limits (< 4,000 ppm). Alternatively, the modified coacervation comprises initially charging the drug, the membrane material and the pore-former into the coacervation tank and following the heating and cooling cycles to produce taste-masked microcapsules with similar properties.

The invention also provides a method of manufacturing orally disintegrating tablets, produced by mixing taste-masked microparticles, rapidly-dispersing microgranules and optionally other excipients (for example: flavor, color, sweetener, additional disintegrant, etc.) to form a blend and compressing the blend into orally disintegrating tablets. In accordance with certain aspects of the invention, the orally disintegrating tablets rapidly disintegrate on contact with saliva in the buccal cavity leaving little or no aftertaste (good creamy mouth feel) and provide rapid, substantially-complete release of the dose in the stomach, thereby enhancing the probability of achieving bioequivalence to the reference product.

Rapidly-dispersing microgranules may be produced in accordance with the method of manufacturing rapidly-dispersing microgranules disclosed in co-pending and commonly assigned U.S. Patent Application No. 10/827,106, filed April 19, 2004. Rapidly dispersing microgranules with an average particle size of 125-300 µm comprising a disintegrant (for example, Crospovidone XL-10) and a sugar alcohol or a saccharide or a mixture thereof (for example, D-mannitol) having an average particle diameter of not more than about 30 *µ*m, may be produced by granulating with only water in a high-shear granulator, wet milling and drying in fluid bed equipment. The taste-masked microparticles produced in accordance with the present invention and rapidly-dispersible microgranules may be blended with other pharmaceutically acceptable ingredients and compressed into tablets, which rapidly disintegrate (e.g., less than about 120 seconds, more particularly less than about 60 seconds) in the buccal cavity with a smooth creamy mouth feel.

In yet another embodiment of the invention, a method to manufacture orally disintegrating tablets is provided. The orally disintegrating tablets may be formed by compressing in a tablet press equipped with an externally lubricating system to pre-lubricate dies and punches and the tablet formulation otherwise being free of lubricant. The orally disintegrating tablets thus produced typically exhibit sufficient hardness and sufficiently low friability and are suitable for packaging in HDPE bottles and push-through blister packs using conventional equipment for storage, transportation and commercial distribution.

The pharmaceutical taste-masked multi-particulate composition in accordance with certain embodiments will provide acceptable taste-masking when placed in the mouth until swallowed (target specification: not more than about 10% of the dose released in about 3 minutes when tested for dissolution in simulated saliva fluid at pH of about 7.0). If the composition is in the ODT (orally disintegrating tablet) form, the tablet typically will disintegrate on contact with saliva in the buccal cavity in about 60 seconds forming a smooth, easy-to swallow suspension, comprising taste-masked microparticles with acceptable aftertaste. These taste-masked microparticles will typically provide substantially-complete release of the dose on entry into the stomach (target specification: not less than about 60%, more particularly not less than 75% of the dose released in about 30 minutes when tested for dissolution in simulated gastric fluid or 0.1N HCl at pH 1.2).

In accordance with one aspect of the invention, a method of manufacturing a taste-masked microparticle composition of one or more bitter-tasting therapeutic agent(s), which exhibits acceptable taste-masking when placed in the oral cavity and provides rapid-release of the dose on entry into the stomach, comprises the following steps:
(a) preparing a drug-containing core particle (crystal with a particle size in the range of about 20-500 µm, bead, pellet or granule) by (i) drug-layering on an inert particle (50-100 mesh sugar sphere or cellulose sphere (e.g., Celphere® CP-203 available from Asahi Kasei Chemicals Corporation)) from a solution/suspension comprising a polymeric binder and the drug in a fluid-bed coater and optionally coating with a seal-coat (e.g., Opadry® Clear), or (ii) granulating the drug and a filler/diluent such as lactose, mannitol or microcrystalline cellulose with a polymeric binder in a high-shear granulator, or (iii) granulating as above, followed by extrusion and spheronization; and
(b) taste-masking core particles by solvent coacervation (microencapsulation) with a mixture of a water-insoluble functional polymer and a gastrosoluble organic or inorganic pore-former (for example, ethylcellulose/ calcium carbonate at a ratio ranging from about 50/50 to 95/5 for a weight gain of about 5% to 50%) to produce effectively taste-masked microparticles with a desired particle size distribution (an average particle size of not more than about 400 *µ*m, more particularly not more than about 300 *µ*m).

In accordance with another aspect of the invention, the method of manufacturing orally disintegrating tablets, which disintegrate on contact with saliva in the buccal cavity forming a smooth, easy-to swallow suspension with acceptable aftertaste, comprising taste-masked microparticles, which rapidly release the dose on entry into the stomach, comprises the following steps:
(a) preparing a drug-containing core particle (crystal with a particle size in the range of about 20-500 µm, bead, pellet or granule) by (i) drug-layering on an inert particle (50-100 mesh sugar sphere or cellulose sphere, e.g., Celphere® CP-203) from a solution/suspension comprising a polymeric binder and the drug in a fluid-bed coater and optionally applying with a seal-coat (e.g., Opadry® Clear), or (ii) granulating the drug and a diluent/filler such as lactose, mannitol or microcrystalline cellulose with a polymeric binder in a high-shear granulator, or (iii) granulating as above, followed by extrusion and spheronization;
(b) taste-masking core particles by solvent coacervation (microencapsulation) of a mixture of a water-insoluble functional polymer and a gastrosoluble pore-former (e.g., ethylcellulose/calcium carbonate at a ratio ranging from about 50/50 to 95/5) to produce pleasant-tasting microparticles with a desired particle size distribution (an average particle size of not more than about 400 *µ*m, more particularly not more than about 300 *µ*m);
(c) granulating a sugar alcohol or a saccharide, or a combination thereof, each of which has an average particle diameter of not more than about 30 *µ*m, with a disintegrant such as Crospovidone using water or an alcohol-water mixture in a typical granulator and drying in fluid-bed equipment to produce rapidly-dispersing microgranules with an average particle size of not more than about 400 *µ*m (typically the average particle size will be in the range of about 100-300 *µ*m);
(d) blending taste-masked microparticles of step (b) with rapidly-dispersing microgranules of step (c) and optionally other pharmaceutically acceptable ingredients such as a flavoring agent, a coloring agent, a sweetener and/or additional disintegrant in sufficient quantities; and
(e) compressing into tablets using a conventional rotary tablet press equipped with an external lubrication system to pre-lubricate the dies and punches.

*In vitro* dissolution testing: The taste-masking property of the taste-masked microparticles and the orally disintegrating tablets may be evaluated in the mouth by determining the percentage of drug-release (a release of not more than about 10% of the dose in about 3 minutes is considered acceptable) when tested for dissolution using USP Apparatus 1 (baskets @ 100 rpm) or 2 (paddles @ 50 rpm) in 900 mL of saliva-simulating fluid (at a pH of about 7.0). Further, the rapid-release property in the stomach of the taste-masked microparticles and the orally disintegrating tablets may be evaluated by determining the percentage of drug-release (a release of not less than about 75% of the dose in about 30 minutes is considered acceptable) when tested for dissolution using USP Apparatus 1 (baskets @ 100 rpm) or Apparatus 2 (paddles @ 50 rpm) in 900 mL of 0.1N HCl (at pH 1.2).

In accordance with certain embodiments of the invention, the taste-masked pharmaceutical composition is in the form of a tablet and exhibits low friability in order to be suitable for packaging blisters and bottles for storage, transportation and commercial distribution. Friability can be determined in accordance with the standard pharmaceutical test methods that are well known to those skilled in the art. Friability for tablets produced in accordance with certain aspects of the invention will have a friability of not more than about 1% and in accordance with certain embodiments not more than about 0.5%.

Examples of therapeutic agents indicated for oral administration suitable for use in accordance with particular embodiments include, but are not limited to, agents such as ranitidine or famotidine (histamine H₂-receptor antagonist), cetirizine or fexofenadine (histamine H₁-receptor antagonist), sumatriptan, electriptan or zolmitriptan (5-HT₁ receptor agonist), ondansetron or granisetron (5-HT₃ receptor antagonist), tiagabine (antiepileptic drug), tizanidine (centrally acting adrenergic agonist), zolpidem or zaleplon (sleep-aid), zafirlukast or montelukast (leukotriene receptor antagonist), and sildenafil or tadalafil (drug for the treatment of erectile dysfunction) requiring taste-masking.

In accordance with particular embodiments, the method of preparing a taste-masked multi-particulate composition includes layering a pharmaceutically acceptable drug from a polymeric binder solution onto an inert particle selected from the group consisting of sugar spheres and cellulose spheres. Fluid bed or pan coating may be used to apply the active and polymeric binder solution.

In accordance with certain embodiments, the core particles may be crystals with a desired particle size distribution, or beads, microgranules or pellets containing one or more active pharmaceutical ingredient(s), requiring taste-masking.

The taste-masked multiparticulate pharmaceutical composition may include a drug-containing core particle that is a drug-layered bead comprising an inert particle such as a sugar sphere, a cellulose sphere or a silicon dioxide sphere coated with one or more pharmaceutically acceptable actives from a polymeric binder solution.

In accordance with certain embodiments, the drug-containing particle is a microgranule or an extruded/spheronized pellet comprising one or more pharmaceutically acceptable active ingredient(s), a polymeric binder, which imparts resilient characteristics to dried microgranules, a hydrophilic filler/diluent, and optionally a flavor, a sweetener and/or a disintegrant.

The microgranules of one or more active pharmaceutical ingredient(s) may be prepared by a conventional high-shear or planetary granulation process or the pellets may be prepared by a conventional granulation-extrusion-spheronization process comprising an active pharmaceutical ingredient, a polymer binder and one or more fillers/diluents.

The water-insoluble polymer (e.g., ethylcellulose with an average viscosity of 100 cps) and the gastrosoluble inorganic pore-former (e.g., calcium carbonate or magnesium oxide) may be present at a weight ratio of from about 95/5 to 50/50, more particularly from about 85/15 to 65/35 and the membrane thickness may vary from about 10% to 30% by weight in accordance with particular embodiments.

In accordance with some embodiments of the present invention, the taste-masked multiparticulate ODT formulation includes rapidly-dispersing microgranules at about 50% to about 90% by weight of the tablet comprising a disintegrant (e.g., crospovidone) and a sugar alcohol (e.g., mannitol) or a saccharide (e.g., lactose) or a combination thereof, each sugar alcohol or saccharide having an average particle diameter of not more than about 30 *µ*m and a ratio of disintegrant to sugar alcohol or saccharide varying from about 90/10 to about 99/1.

In accordance with some other embodiments of the present invention, the rapidly-dispersing microgranules and taste-masked microparticles may be present in the ratio of about 6/1 to 2/1 to provide taste-masked composition having a smooth mouth feel.

In accordance with certain embodiments of the present invention, a method of manufacturing a taste-masked multi-particulate composition of one or more active pharmaceutical ingredients is also provided. The method may comprise the steps of:
(a) preparing core particles (crystals with a particle size distribution of about 20-500 µm, more particularly of about 30-300 µm, beads, microgranules or pellets) of one or more active pharmaceutical ingredient(s) (i) as beads by drug-layering onto inert particles from a polymeric binder solution in fluid-bed equipment, (ii) as microgranules by conventional granulation of one or more active pharmaceutical ingredient(s), one or more polymeric binders, a hydrophilic filler/diluent, and optionally a flavor, a sweetener, and /or a disintegrant, or (iii) as pellets by a granulation-extrusion-spheronization process; and
(b) microencapsulating core particles by solvent coacervation with a mixture of a water-insoluble polymer and a gastrosoluble pore-former at a ratio of about 95/5 to 50/50, the membrane coating comprising from about 5% to about 50% based on the total weight of the coated particles.

The composition may exhibit the following properties in certain embodiments:
1. acceptable taste-masking when the composition is placed in the oral cavity for 3 minutes, more particularly for 2 minutes and in certain embodiments for 60 seconds, and in still other embodiments, until it is swallowed leaving no aftertaste; and
2. rapid, substantially-complete release of the dose upon entry into the stomach, i.e., releases not less than about 75% of the dose in 30 minutes when tested for dissolution using United States Pharmacopoeia Apparatus 1 (Baskets @ 100 rpm in 900 mL of pH 1.2 buffer).

In accordance with certain embodiments, a method of preparing an ODT composition comprises the steps of:
(a) preparing core particles (crystals with a particle size distribution of about 20-500 µm, more particularly of about 30-300 µm, beads, microgranules or pellets) of one or more active pharmaceutical ingredient(s) as described above;
(b) microencapsulating core particles by solvent coacervation with a mixture of a water-insoluble polymer and a gastrosoluble pore-former at a ratio of about 95/5 to 50/50, the membrane coating comprising from about 5% to 50% based on the total weight of the coated particles;
(c) granulating a disintegrant such as crospovidone with a sugar alcohol or a saccharide, or a combination thereof, each having an average particle diameter of not more than about 30 *µ*m, with water or an alcohol-water mixture in a conventional granulator and drying in fluid bed equipment to produce granules with an average particle size not more than about 400 *µ*m (more particularly not more than about 300 *µ*m);
(d) blending taste-masked microparticles of step (b) with rapidly disintegrating microgranules of step (c) at a ratio of about 1/6 to about 1/2, and optionally other, pharmaceutically acceptable ingredients, such as a flavoring agent (<0.5% w/w), a coloring agent (<0.5% w/w), a sweetener (<0.5% w/w) and additional disintegrant (up to 4% w/w); and
(e) compressing into tablets using a conventional rotary tablet press equipped with an external lubrication system to pre-lubricate the dies and punches.

The ODT may exhibit the following properties :
1) disintegrates on contact with saliva in the oral cavity forming a smooth, easy-to-swallow suspension comprising taste-masked microparticles;
2) leaves no aftertaste after swallowed (no gritty or chalky mouthfeel);
3) provides rapid, substantially-complete release of the dose upon entry into the stomach; or
4) the ODT when tested for dissolution using United States Pharmacopoeia Apparatus 1 (baskets @ 100 rpm in 900 mL buffer) releases not more than about 10% of the dose in about 3 minutes in a simulated saliva buffer at pH 6.8 and not less than about 75% of the dose in about 30 minutes in an acidic buffer at pH 1.2.

The following non-limiting examples illustrate the taste-masked microparticle composition or an orally disintegrating tablet dosage form comprising one or more therapeutic agent(s) requiring taste-masking, manufactured in accordance with the invention, which exhibits acceptable taste-masking when placed in the mouth and substantially complete, rapid-release of the dose on entry into the stomach.

### Example 1 - Inventive

The Cetirizine Microgranules (drug load: approximately 20% cetirizine hydrochloride): Cetirizine hydrochloride (20%), microcrystalline cellulose (70%) and hydroxypropyl methylcellulose (Methocel K100LV at 10% by weight) were granulated with purified water in a high-shear granulator and dried in a tray-drying oven.

Taste-masked Microgranules (drug load: approximately 12.2% cetirizine hydrochloride): Microgranules (700 g) with a low friability obtained above were microencapsulated using the improved solvent coacervation process. Ethocel (ethylcellulose) Standard 100 Premium (100 cps), from Dow Chemicals (300 g) was dissolved in a 5-gallon coacervation tank at 80°C. The micronized pore-former (150 g calcium carbonate) was added into the coacervation tank at a product temperature of approximately 58°C during the temperature-programmed cooling cycle to achieve a uniform distribution of the pore-former throughout the ethylcellulose membrane. Upon reaching the ambient temperature, the microcapsules with a membrane coating of 2/1 ethylcellulose/calcium carbonate at approximately 39% by weight were filtered, washed with fresh cyclohexane and dried to reduce the residual solvent level to within acceptable limits. The taste-masked microparticles with an average particle size of 230 µm had an acceptable taste.

Rapidly Dispersing Microgranules: The rapidly dispersing microgranules may comprise a sugar alcohol such as mannitol and/or a saccharide such as lactose and a disintegrant such as Crospovidone. The sugar alcohol and/or saccharide and disintegrant will typically be present in the rapidly dispersing microgranules at a ratio of from about 99:1 to about 90:10 (sugar alcohol and/or saccharide:disintegrant). For example, D-mannitol, a sugar alcohol with an average particle size of about 15 µm and Crospovidone XL-10, a super disintegrant, may be used at a ratio of about 90/10 in a high shear granulator using purified water as the granulating fluid.

Cetirizine Hydrochloride ODT, 10 mg (as cetirizine hydrochloride): 81 mg of taste-masked microparticles and 529 mg of rapidly-dispersing microgranules were blended with 32.5 mg of crospovidone, 6.5 mg of an orange flavor, 0.65 mg of Sucralose (a sweetener) and compressed into tablets (13mm (diameter) x 4.68mm) with an average weight of 650 mg and average hardness of 97 N and friability of 0.5% to demonstrate robustness of the manufacturing (taste-masking and tableting) process and meeting target dissolution specifications (not more than about 10% in 5 minutes in the simulated saliva fluid at pH 6.8 and not less than about 75% released in 30 minutes in 0.1N HCl).

### Example 2 - Comparative

Cetirizine Microgranules (drug load: approximately 20% cetirizine hydrochloride): Cetirizine HCl (20%), microcrystalline cellulose (70%) and hydroxypropyl methylcellulose (Methocel K100LV at 10% by weight) were granulated with water in a high-shear granulator and dried in a tray-drying oven.

Taste-masked Microgranules (drug load: approximately 14% cetirizine hydrochloride): Microgranules with a low friability obtained above were taste-masked by solvent coacervation with 100% ethylcellulose as described in Example 1 except that no micronized calcium carbonate was added during the temperature-programmed cooling cycle. The ethylcellulose coating level was approximately 30% by weight.

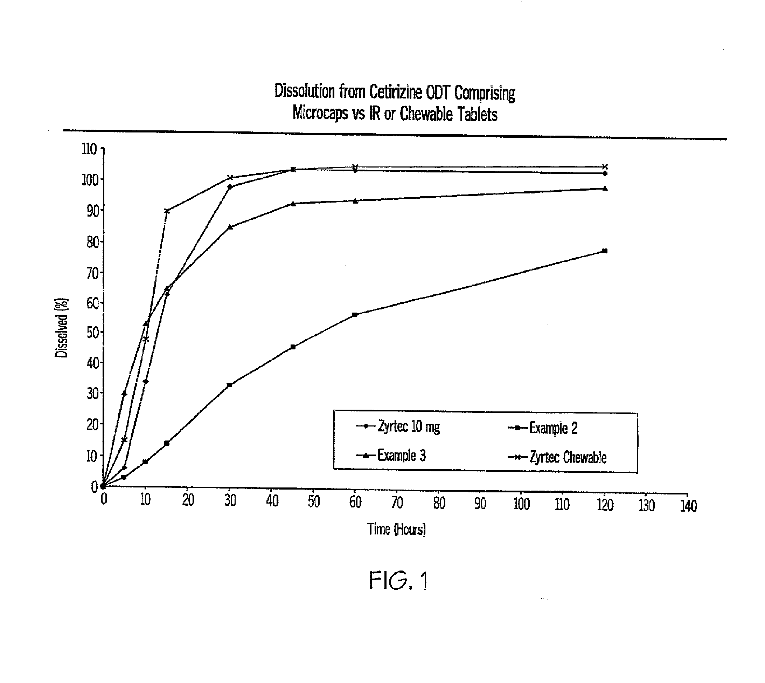

Cetirizine Hydrochloride ODT, 10 mg (as cetirizine hydrochloride 71 mg of taste-masked microparticles and 542.6 mg of rapidly-dispersing microgranules were blended with crospovidone (32.5 mg), an orange flavor (3.25 mg), Sucralose (0.65 mg) and compressed into tablets with an average weight of 650 mg and average hardness of 97 N to demonstrate robustness of the manufacturing (taste-masking and tableting) process and meeting target dissolution specifications when dissolution tested using USP Apparatus 2 (50 rpm) in 0.1N HCl. The dissolution profiles are presented in Fig. 1.

### Example 3 - Inventive

Cetirizine Microgranules (drug load: approximately 20% cetirizine hydrochloride): Cetirizine hydrochloride (20%), microcrystalline cellulose (70%) and hydroxypropyl methylcellulose (Methocel Kl00LV at 10% by weight) were granulated with water in a high-shear granulator and dried in a tray-drying oven.

Taste-masked Microgranules (drug load: approximately 12.2% cetirizine hydrochloride): Microgranules with a low friability obtained above were taste-masked by solvent coacervation with 2/1 ethylcellulose/calcium carbonate (micronized) as described in Example 1.

Cetirizine Hydrochloride ODT, 10 mg (as cetirizine Hydrochloride): 82 g of taste-masked microparticles and 531.6 g of rapidly-dispersing microgranules were blended with crospovidone (32.5 mg), an orange flavor (3.25 g), Sucralose (0.65 g) and compressed into tablets with an average weight of 650 mg and average hardness of 97 N to demonstrate robustness of the manufacturing (taste-masking and tableting) process and meeting target dissolution specifications when dissolution tested using USP Apparatus 2 (50 rpm) in 0.1N HCI+0.01%Tween 80.

Dissolution Testing of ODTs of Example 2 and 3: The tablets of Example 2 and 3 along with commercially-available products, Zyrtec IR Tablets, 10 mg and Zyrtec Chewable Tablets, 10 mg were dissolution tested using USP Apparatus 2 in Purified Water USP and HPLC. The dissolution profiles are presented in Fig. 1.

### Example 4

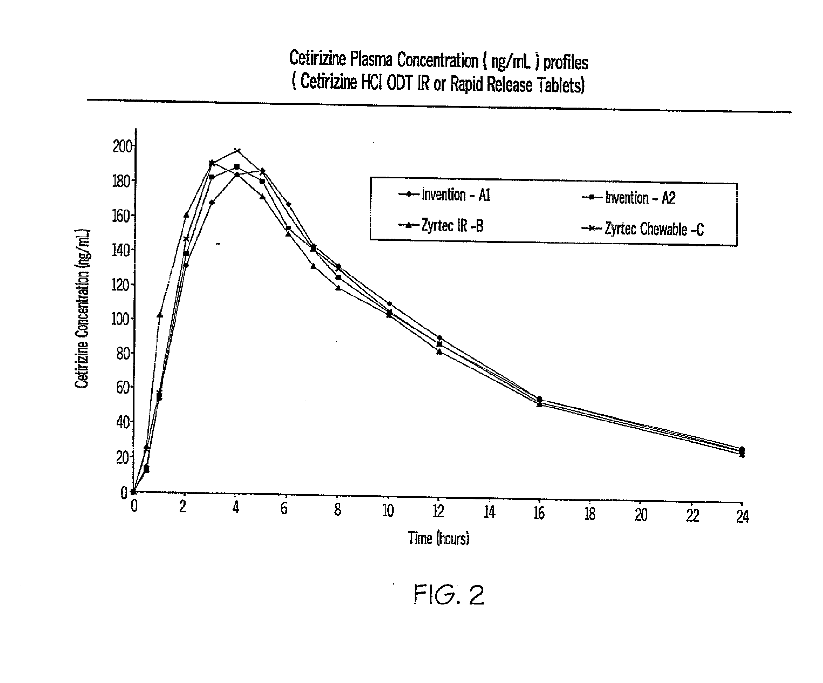

Pilot PK Study in Humans: A 4-arm, randomized pilot PK (pharmacokinetics) study was conducted in 12 healthy adult subjects dosing (A1) one 10 mg ODT sample prepared in accordance with the present invention (Example 3) with water (designated as Invention-Al in Fig. 2), (A2) one 10 mg ODT sample prepared in accordance with the present invention (Example 3) without water (designated as Invention-A2 in the Fig. 2), (B) one 10 mg Zyrtec IR Tablet with water, or (C) one 10 mg Zyrtec Chewable Tablet with water. Blood samples were withdrawn at 0, 0.5, 1, 2, 3, 4, 5, 6, 7, 8, 10, 12, 16, and 24 hour time points and plasma concentrations were determined bioanalytically. The plasma concentration profiles are presented in Fig. 2. The PK parameters are presented in Table 1. The ODT formulations administered with and without water were judged to be bioequivalent to both Zyrtec IR or Chewable tablets.

**Table 1: Pilot PK Data for Cetirizine**

| PK Parameter | ODT with Water (A1) | ODT w/o Water (A2) | Zyrtec IR Tablet with Water (B) | Zyrtec Chewable with Water (C) |
|---|---|---|---|---|
| AUC_{0.24 hr} | | | | |
| (ng-hr/mL) | | | | |
| AUC_{0-24 hr} | A1/A2: | | A1/B: | A1/C: |
| 90% Confidence Interval (CI) | 0.9692-1.0779 | | 0.9806-1.0589 | 0.9581-1.0379 |
| | | | A2/B: | A2/C: |
| | | | 0.9267-1.0668 | 0.9107-1.0452 |
| Mean Cmax | 187.44 | 189.15 | 191.40 | 198.14 |
| (ng/mL) | | | | |
| Cₘₐₓ | A1/A2: | | A1/B: | A1/C: |
| 90% CI | 0.9061-1.0158 | | 0.8531- 0.9718 | 0.8994-1.0005 |
| | | | A2/B: | A2/C: |
| | | | 0.8809 -1.0225 | 0.9342- 1.0456 |
| Tₘₐₓ | 4.0 hr | 4.0 hr | 4.0 hr | 4.0 hr |

### Example 5 - Comparative

Sumatriptan Succinate Microgranules (drug load: approximately 90% as salt): Sumatriptan succinate (90%) and hydroxypropyl methylcellulose (Methocel K100LV at 10% by weight) were granulated with water in a high-shear granulator and dried in a tray-drying oven. These microgranules exhibited a low friability.

Taste-masked Sumatriptan Succinate (drug load: approximately 54%): The microgranules obtained above were taste-masked by solvent coacervation with ethylcellulose alone for a weight gain of approximately 40% as in Example 2.

Sumatriptan Succinate ODT, 100 mg (as sumatriptan base): 259 mg of coated microparticles taste-masked with ethylcellulose alone and 845 mg of rapidly-dispersing microgranules were blended with crospovidone (60 mg), an orange flavor (24 mg), Sucralose (12 mg) and compressed into tablets with a force of 1.5 ton and average hardness of 70 N to demonstrate robustness of the manufacturing (taste-masking and tableting) process and meeting target dissolution specifications.

### Example 6 - Inventive

Sumatriptan Succinate Microgranules (drug load: approximately 90%): Sumatriptan succinate (90%) and hydroxypropyl methylcellulose (Methocel K100LV at 10% by weight) were granulated with water following Example 5.

Taste-masked Sumatriptan Succinate (drug load: approximately 55%): Microgranules with a low friability obtained above were taste-masked by solvent coacervation with 2/1 ethylcellulose / calcium carbonate for a weight gain of approximately 45% by weight (or equivalent to 30% ethylcellulose/15% calcium carbonate) as in Example 1.

Sumatriptan Succinate ODT, 100 mg (as sumatriptan): 255 mg of taste-masked microparticles and 849 mg of rapidly-dispersing microgranules were blended with crospovidone (60 mg), an orange flavor (24 mg), Sucralose (12 mg) and compressed into tablets at a force of 1.5 ton and average hardness of 70 N to demonstrate robustness of the manufacturing (taste-masking and tableting) process. Dissolution testing was performed using USP Apparatus 2 (30 rpm) in 0.01N HCl.

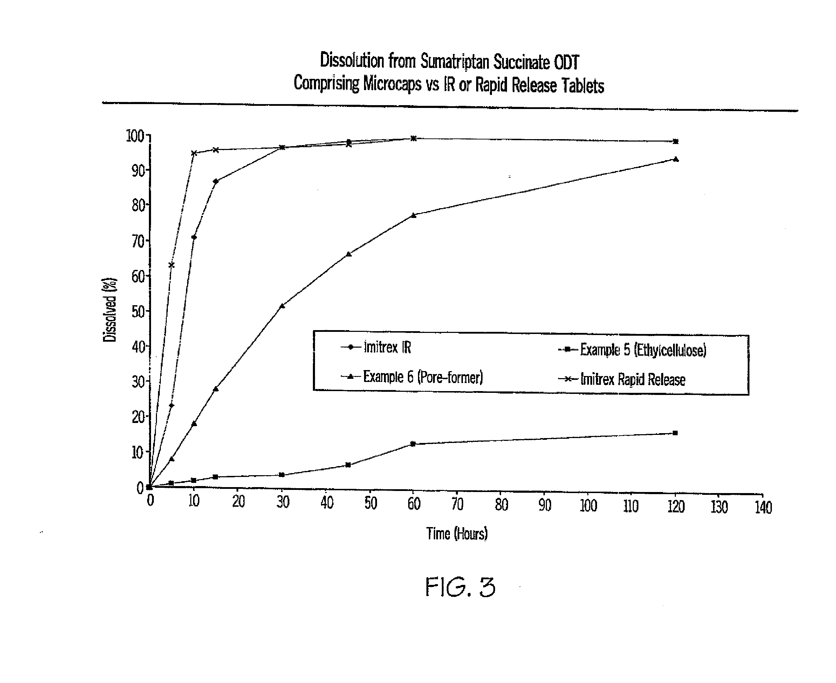

Dissolution Testing of ODTs of Example 5 and 6: Dissolution testing of the tablets of Example 5 and 6 was performed using USP Apparatus 2 (30 rpm) in 0.01N HCl. The dissolution data are given in Fig. 3. The ODT formulation containing the coated microparticles taste-masked with ethylcellulose alone is designated in the legend as Example 5 (Ethylcellulose) while the tablet formulation containing the coated microparticles taste-masked by the pore-former technology is designated as Example 6 (Pore-former).

### Example 7 - Inventive

Cetirizine Microgranules (drug load: approximately 20% cetirizine hydrochloride): Cetirizine HCl (20%), microcrystalline cellulose (70%) and hydroxypropyl methylcellulose (Methocel K100LV at 10% by weight) were granulated with water following Example 2.

Taste-masked Microgranules (drug load: approximately 12.2% cetirizine hydrochloride): Microgranules with a low friability obtained above were taste-masked by solvent coacervation with 2/1 ethylcellulose /calcium carbonate (micronized) as described in Example 3 with the exception that the coacervation was charged with both ethylcellulose and calcium carbonate before heating cyclohexane (i.e., instead of adding calcium carbonate at the product temperature of 60±2°C during the computer controlled cooling cycle).

Dissolution testing: The Microcaps were dissolution tested using USP Apparatus 2 in Purified Water USP. The dissolution data are presented in Table 2. The dissolution data are similar to that obtained for Microcaps of Example 3 suggesting that both ethylcellulose and calcium carbonate can be charged into the coacervation tank at the same time if required.

**Table 2: Dissolution Data for Cetirizine Hydrochloride Tablets of Example 7**

| **Time (minutes)** | **% Dissolved (Pore-former added with Ethylcellulose)** | **% Dissolved (Pore-former added during Cooling)** |
|---|---|---|
| 0 | 0 | 0 |
| 5 | 9 | 13 |
| 10 | 20 | 29 |
| 15 | 30 | 40 |
| 30 | 52 | 65 |
| 45 | 68 | 78 |
| 60 | 79 | 86 |
| 120 | 97 | 98 |

### Example 8 - Inventive

Taste-masked microparticles of Potassium Chloride (drug load: approximately 80%): 35-50 mesh potassium chloride crystals (35.4kg), Ethocel (6.72 kg), polyethylene (Epolene) as a phase inducer (2.2 kg), and micronized calcium carbonate (2.88 kg) would be suspended in cyclohexane in a 200-gallon coacervation tank. The usual heating procedure was followed to dissolve both Ethocel and Epolene in cyclohexane at 80°C.
Thereafter the temperature-programmed cooling cycle was followed to achieve a uniform distribution of the pore-former throughout the ethylcellulose membrane. Upon reaching ambient temperature, the microcapsules would be filtered, washed with fresh cyclohexane to remove traces of Epolene adhering to the microcapsules and dried to reduce the residual solvent level to within acceptable limits. The taste-masked microparticles would exhibit an acceptable taste and meet target dissolution specifications both in simulated saliva at pH 6.8 and 0.1N HCl at pH 1.2.

Potassium Chloride ODT, 100 mg: The taste-masked microparticles (1,775 g), rapidly-dispersing microgranules (3,100 g) and an orange flavor (15 g), Aspartame (20 g), and crospovidone (90 g) would be blended and compressed into 100 mg tablets weighing 1 g using a Fette tablet press equipped with an external lubricating system. The drug-release would meet target dissolution specifications (not more than about 10% in 5 minutes in the simulated saliva fluid at pH 6.8 and not less than about 75% released in 30 minutes in 0.1N HCl.

Changes may be made by persons skilled in the art in the composition and the manufacturing procedures as described herein or in the steps or the sequence of steps of the method of manufacture described therein without departing from the spirit and scope of the invention as described above.

The invention will now be described by reference to the following clauses:
1. A taste-masked multiparticulate pharmaceutical composition comprising:
   (a) a drug-containing core particle, and
   (b) a solvent-coacervated membrane on said drug-containing core particle comprising a combination of a water-insoluble polymer and a gastrosoluble, organic or inorganic pore-former at a ratio ranging from about 90/10 to about 50/50, said membrane having a thickness of from about 10% to about 50% by weight based on the weight of the coated particle and an average particle size of not more than about 400µm,
      wherein said composition has the following properties:
      (1) said composition has acceptable taste-masking when the composition is placed in the oral cavity for at least about 60 seconds; and
      (2) said composition releases not less than about 75% of the dose in 30 min when tested for dissolution using United States Pharmacopoeia Apparatus 1 (Baskets@ 100 rpm in 900 mL of pH 1.2 buffer).
2. A taste-masked multiparticulate pharmaceutical composition of clause 1 further comprising:
   (c) rapidly-dispersing microgranules with an average particle size of not more than about 300µm comprising: i) a disintegrant and ii) a sugar alcohol, a saccharide or a combination thereof, having an average particle diameter of not more than about 30*µ*m, and
   (d) one or more pharmaceutically acceptable excipient,
      wherein said composition is in the form of a tablet and the tablet exhibits the following properties:
      (1) exhibits a friability of not more than about 1%; and
      (2) disintegrates with the saliva in the oral cavity within approximately 60 seconds.
3. A taste-masked multiparticulate pharmaceutical composition of clause 1 wherein said drug-containing core particle comprises a drug-layered bead comprising an inert particle coated with one or more pharmaceutically acceptable actives from a polymeric binder solution.
4. A taste-masked multiparticulate pharmaceutical composition of clause wherein said drug-containing particle is a microgranule or an extruded/spheronized pellet comprising one or more pharmaceutically acceptable active ingredient(s), a polymeric binder, which imparts resilient characteristics to dried microgranules, a hydrophilic filler/diluent, and optionally a flavor, a sweetener and/or a disintegrant.
5. A taste-masked multiparticulate pharmaceutical composition of clause 2 wherein said composition comprises taste-masked microparticles that release not more than about 10% in about 3 minutes when dissolution tested in a simulated saliva fluid (pH ∼6.7-7.4).
6. A taste-masked multiparticulate pharmaceutical composition of clause1 wherein said drug is a pharmaceutically acceptable active ingredient requiring taste-masking.
7. A taste-masked multiparticulate pharmaceutical composition of clause1 wherein said drug comprises a therapeutic agent indicated for oral administration selected from the group consisting of cetirizine, fexofenadine, sumatriptan, electriptan, zolmitriptan, ondansetron, granisetron, tiagabine, tizanidine, zolpidem, zaleplon, zafirlukast, montelukast, sildenafil, vardenafil, tadalafil, their salts and mixtures thereof.
8. A taste-masked multiparticulate pharmaceutical composition of clause 1 wherein said water insoluble polymer is selected from the group consisting of ethylcellulose, cellulose acetate, cellulose acetate butyrate, polyvinyl acetate, neutral methacrylic ester copolymer, ammonio-methacrylate copolymers and mixtures thereof and said gastrosoluble organic or inorganic pore-former is selected from the group consisting of calcium carbonate, calcium phosphate, calcium saccharide, calcium succinate, calcium tartrate, ferric acetate, ferric hydroxide, ferric phosphate, magnesium carbonate, magnesium citrate, magnesium hydroxide, magnesium phosphate, and mixtures thereof.
9. A taste-masked multiparticulate pharmaceutical composition of clause2 wherein said water insoluble polymer is selected from the group consisting of ethylcellulose, cellulose acetate, cellulose acetate butyrate, polyvinyl acetate, neutral methacrylic ester copolymer, ammonio-methacrylate copolymers and mixtures thereof and said gastrosoluble organic or inorganic pore-former is selected from the group consisting of calcium carbonate, calcium phosphate, calcium saccharide, calcium succinate, calcium tartrate, ferric acetate, ferric hydroxide, ferric phosphate, magnesium carbonate, magnesium citrate, magnesium hydroxide, magnesium phosphate, and mixtures thereof.
10. A taste-masked multiparticulate pharmaceutical composition of clause2 wherein said disintegrant and said sugar alcohol, saccharide or combination thereof are present in said rapidly-dispersing microgranules at a ratio of disintegrant to sugar alcohol, saccharide or combination thereof at from about 90/10 to about 99/1.
11. A taste-masked multiparticulate pharmaceutical composition of clause2 wherein said rapidly-dispersing microgranules and taste-masked microparticles are present a ratio of from about 5/1 to 1/1, respectively.
12. A taste-masked multi-particulate composition of clause2 in an ODT (orally disintegrating tablet) form wherein said rapidly-dispersing microgranules comprise a disintegrant selected from the group consisting of crospovidone, sodium starch glycolate, crosslinked carboxymethyl cellulose of sodium, low-substituted hydroxylpropylcellulose and mixtures thereof and a sugar alcohol or saccharide selected from the group consisting of mannitol, xylitol, sorbitol, maltol, maltitol, lactose, sucrose, maltose and combinations thereof.
13. A method of manufacturing a taste-masked multi-particulate composition of one or more active pharmaceutical ingredients, wherein said method comprises the steps of:
   a. preparing core particles of one or more active pharmaceutical ingredient(s) as beads by drug-layering onto inert particles from a polymeric binder solution in a fluid-bed equipment, or as microgranules by a high-shear or planetary granulation process of one or more active pharmaceutical ingredient(s), one or more polymeric binder(s), a hydrophilic filler/diluent, and optionally a flavor, a sweetener, and /or a disintegrant or as pellets by granulation-extrusion-spheronization process; and
   b. applying on the core particles a membrane comprising a mixture of a water-insoluble polymer and a gastrosoluble organic or inorganic pore-former at a ratio of about 90/10 to 50/50 by temperature-induced phase separation in an organic solvent system, said membrane coating comprising approximately from about 10% to 50% based on the total weight of the coated particles;
      wherein said composition has the following properties:
      (1) said composition has acceptable taste-masking when the composition is placed in the oral cavity for at least about 60 seconds; and
      (2) said composition releases not less than about 75% of the dose in about 30 min when tested for dissolution using United States Pharmacopoeia Apparatus 1 (Baskets@ 100 rpm in 900 mL of pH 1.2 buffer).
14. A method of manufacturing a taste-masked multi-particulate composition of one or more active pharmaceutical ingredients in accordance with clause 13, wherein said method further comprises the steps of:
   c. granulating a disintegrant with a sugar alcohol, a saccharide, or a combination thereof, each having an average particle diameter of not more than about 30 *µ*m, with water or an alcohol-water mixture to produce granules with an average particle size of not more than about 400 *µ*m;
   d. blending taste-masked microparticles of step (b) with rapidly disintegrating microgranules of step (c) and optionally other acceptable ingredients; and
   e. compressing into tablets using a tablet press equipped with an external lubrication system to pre-lubricate the dies and punches.
      wherein said composition exhibits at least one of the following properties:
      (1) disintegrates with the saliva in the oral cavity;
      (2) leaves no aftertaste after swallowed;
      (3) provides rapid, substantially-complete release of the dose upon entry into the stomach; or
      (4) wherein said composition when tested for dissolution using United States Pharmacopoeia Apparatus 1 (baskets@ 100 rpm in 900 mL buffer) releases not more than about 10% of the dose in about 3 minutes in a simulated saliva buffer at pH 6.8.
15. A method of manufacturing taste-masked multi-particulate composition in accordance with clause14 in the orally disintegrating tablet (ODT) form, wherein said rapidly-dispersing microgranules comprising a disintegrant selected from the group consisting of crospovidone, sodium starch glycolate, crosslinked carboxymethyl cellulose of sodium, and low-substituted hydroxylpropylcellulose and a sugar alcohol or saccharide selected from the group consisting of mannitol, xylitol, sorbitol, maltol, maltitol, lactose, sucrose, maltose and combinations thereof, each having an average particle diameter of not more than about 30*µ*m.
16. A method of manufacturing taste-masked multi-particulate composition in accordance with clause 13 wherein said drug-containing core particles are coated with a blend of water-insoluble ethycellulose and calcium carbonate at a ratio of from about 90/10 to about 50/50 for a weight gain of not less than about 10% and not more than about 50%, based on the total weight of the coated particle.
17. A method of preparing a taste-masked multi-particulate composition in accordance with clause13 wherein drug-containing beads are prepared by layering a pharmaceutically acceptable drug from a polymeric binder solution onto sugar spheres or cellulose spheres in fluid-bed equipment.
18. A method of preparing a taste-masked multi-particulate composition in accordance with clause 13 wherein said microgranules of one or more active pharmaceutical ingredient(s) are prepared by a high shear or planetary granulation process or said pellets are prepared by a granulation-extrusion-spheronization process comprising an active pharmaceutical ingredient, a polymer binder and one or more fillers/diluents.
19. A method of preparing a taste-masked multi-particulate composition in accordance with clause 13 wherein said water insoluble polymer is selected from the group consisting of ethylcellulose, cellulose acetate, cellulose acetate butyrate, polyvinyl acetate, ammonio-methacrylate copolymers and mixtures thereof, and said gastrosoluble organic or inorganic pore-former is selected from the group consisting of calcium carbonate, calcium phosphate, calcium saccharide, calcium succinate, calcium tartrate, ferric acetate, ferric hydroxide, ferric phosphate, magnesium carbonate, magnesium citrate, magnesium hydroxide, magnesium phosphate, and mixtures thereof.
20. A method of manufacturing a taste-masked multi-particulate composition clause 13 wherein said dosage form comprises one or more active pharmaceutical ingredients selected from the group consisting of histamine H₂ receptor antagonists, proton pump inhibitors, 5-HT₃ agonists, histamine H₁ receptor antagonists, antiepileptic drugs, centrally acting adrenergic agonists, sleep aids, drugs for the treatment of erectile dysfunction, salts thereof and mixtures thereof.
21. A method of manufacturing a taste-masked multi-particulate composition clause 13 wherein said dosage form comprises one or more active pharmaceutical ingredients in sufficient quantities to be administered orally to a patient at prescribed dosing regimen to provide therapeutic efficacy.

## Claims

1. A taste-masked multiparticulate pharmaceutical composition comprising:
(a) a drug-containing core particle, and
on said drug-containing core particle comprising a combination of a water-insoluble polymer and a gastrosoluble, organic or inorganic pore-former at a ratio ranging from about 90/10 to about 50/50, said membrane having a thickness of from about 10% to about 50% by weight based on the weight of the coated particle and an average particle size of not more than about 400 µm, wherein said composition has the following properties:
(1) said composition has acceptable taste-masking when the composition is placed in the oral cavity for at least about 60 seconds; and
(2) said composition releases not less than about 75% of the dose in 30 min when tested for dissolution using United States Pharmacopoeia Apparatus 1 (Baskets@100 rpm in 900 mL of pH 1.2 buffer).

2. A taste-masked multiparticulate pharmaceutical composition of claim 1 further comprising:
(c) rapidly-dispersing microgranules with an average particle size of not more than about 300 µm comprising: i) a disintegrant and ii) a sugar alcohol, a saccharide or a combination thereof, having an average particle diameter of not more than about 30 µm, and
(d) one or more pharmaceutically acceptable excipient,
wherein said composition is in the form of a tablet and the tablet exhibits the following properties:
(1) exhibits a friability of not more than about 1%; and
(2) disintegrates with the saliva in the oral cavity within approximately 60 seconds.

3. A taste-masked multiparticulate pharmaceutical composition of claim 1 wherein said drug-containing core particle comprises a drug-layered bead comprising an inert particle coated with one or more pharmaceutically acceptable actives from a polymeric binder solution.

4. A taste-masked multiparticulate pharmaceutical composition of claim 1 wherein said drug-containing particle is a microgranule or an extruded/spheronized pellet comprising one or more pharmaceutically acceptable active ingredient(s), a polymeric binder, which imparts resilient characteristics to dried microgranules, a hydrophilic filler/diluent, and optionally a flavor, a sweetener and/or a disintegrant.

5. A taste-masked multiparticulate pharmaceutical composition of claim 2 wherein said composition comprises taste-masked microparticles that release not more than about 10% in about 3 minutes when dissolution tested in a simulated saliva fluid (pH ∼6.7-7.4).

6. A taste-masked multiparticulate pharmaceutical composition of claim 1 wherein said drug is a pharmaceutically acceptable active ingredient requiring taste-masking.

7. A taste-masked multiparticulate pharmaceutical composition of claim 1 wherein said drug comprises a therapeutic agent indicated for oral administration selected from the group consisting of cetirizine, fexofenadine, sumatriptan, electriptan, zolmitriptan, ondansetron, granisetron, tiagabine, tizanidine, zolpidem, zaleplon, zafirlukast, montelukast, sildenafil, vardenafil, tadalafil, their salts and mixtures thereof.

8. A taste-masked multiparticulate pharmaceutical composition of claim 1 wherein said water insoluble polymer is selected from the group consisting of ethylcellulose, cellulose acetate, cellulose acetate butyrate, polyvinyl acetate, neutral methacrylic ester copolymer, ammonio-methacrylate copolymers and mixtures thereof and said gastrosoluble organic or inorganic pore-former is selected from the group consisting of calcium carbonate, calcium phosphate, calcium saccharide, calcium succinate, calcium tartrate, ferric acetate, ferric hydroxide, ferric phosphate, magnesium carbonate, magnesium citrate, magnesium hydroxide, magnesium phosphate, and mixtures thereof.

9. A taste-masked multiparticulate pharmaceutical composition of claim 2 wherein said water insoluble polymer is selected from the group consisting of ethylcellulose, cellulose acetate, cellulose acetate butyrate, polyvinyl acetate, neutral methacrylic ester copolymer, ammonio-methacrylate copolymers and mixtures thereof and said gastrosoluble organic or inorganic pore-former is selected from the group consisting of calcium carbonate, calcium phosphate, calcium saccharide, calcium succinate, calcium tartrate, ferric acetate, ferric hydroxide, ferric phosphate, magnesium carbonate, magnesium citrate, magnesium hydroxide, magnesium phosphate, and mixtures thereof.

10. A taste-masked multiparticulate pharmaceutical composition of claim 2 wherein said disintegrant and said sugar alcohol, saccharide or combination thereof are present in said rapidly-dispersing microgranules at a ratio of disintegrant to sugar alcohol, saccharide or combination thereof at from about 90/10 to about 99/1,
and/or wherein said rapidly-dispersing microgranules and taste-masked microparticles are present a ratio of from about 5/1 to 1/1, respectively,
and/or wherein, in an ODT (orally disintegrating tablet) form, said rapidly-dispersing microgranules comprise a disintegrant selected from the group consisting of crospovidone, sodium starch glycolate, crosslinked carboxymethyl cellulose of sodium, low-substituted hydroxylpropylcellulose and mixtures thereof and a sugar alcohol or saccharide selected from the group consisting of mannitol, xylitol, sorbitol, maltol, maltitol, lactose, sucrose, maltose and combinations thereof.

11. A method of manufacturing a taste-masked multi-particulate composition of one or more active pharmaceutical ingredients, wherein said method comprises the steps of:
a. preparing core particles of one or more active pharmaceutical ingredient(s) as beads by drug-layering onto inert particles from a polymeric binder solution in a fluid-bed equipment, or as microgranules by a high-shear or planetary granulation process of one or more active pharmaceutical ingredient(s), one or more polymeric binder(s), a hydrophilic filler/diluent, and optionally a flavor, a sweetener, and /or a disintegrant or as pellets by granulation-extrusion-spheronization process; and
b. applying on the core particles a membrane comprising a mixture of a water-insoluble polymer and a gastrosoluble organic or inorganic pore-former at a ratio of about 90/10 to 50/50 by temperature-induced phase separation in an organic solvent system, said membrane coating comprising approximately from about 10% to 50% based on the total weight of the coated particles; wherein said composition has the following properties:
(1) said composition has acceptable taste-masking when the composition is placed in the oral cavity for at least about 60 seconds; and
(2) said composition releases not less than about 75% of the dose in about 30 min when tested for dissolution using United States Pharmacopoeia Apparatus 1 (Baskets@100 rpm in 900 mL of pH 1.2 buffer).

12. A method of manufacturing a taste-masked multi-particulate composition of one or more active pharmaceutical ingredients in accordance with claim 11, wherein said method further comprises the steps of:
c. granulating a disintegrant with a sugar alcohol, a saccharide, or a combination thereof, each having an average particle diameter of not more than about 30 µm, with water or an alcohol-water mixture to produce granules with an average particle size of not more than about 400 µm;
d. blending taste-masked microparticles of step (b) with rapidly disintegrating microgranules of step (c) and optionally other acceptable ingredients; and
e. compressing into tablets using a tablet press equipped with an external lubrication system to pre-lubricate the dies and punches. ,
wherein said composition exhibits at least one of the following properties:
(1) disintegrates with the saliva in the oral cavity;
(2) leaves no aftertaste after swallowed;
(3) provides rapid, substantially-complete release of the dose upon entry into the stomach; or
(4) wherein said composition when tested for dissolution using United States Pharmacopoeia Apparatus 1 (baskets@100 rpm in 900 mL buffer) releases not more than about 10% of the dose in about 3 minutes in a simulated saliva buffer at pH 6.8,
particularly wherein, in the orally disintegrating tablet (ODT) form, said rapidly-dispersing microgranules comprising a disintegrant selected from the group consisting of crospovidone, sodium starch glycolate, crosslinked carboxymethyl cellulose of sodium, and low-substituted hydroxylpropylcellulose and a sugar alcohol or saccharide selected from the group consisting of mannitol, xylitol, sorbitol, maltol, maltitol, lactose, sucrose, maltose and combinations thereof, each having an average particle diameter of not more than about 30 µm.

13. A method of manufacturing taste-masked multi-particulate composition in accordance with claim 11 wherein said drug-containing core particles are coated with a blend of water-insoluble ethycellulose and calcium carbonate at a ratio of from about 90/10 to about 50/50 for a weight gain of not less than about 10% and not more than about 50%, based on the total weight of the coated particle,
and/or wherein said water insoluble polymer is selected from the group consisting of ethylcellulose, cellulose acetate, cellulose acetate butyrate, polyvinyl acetate, ammonio-methacrylate copolymers and mixtures thereof, and said gastrosoluble organic or inorganic pore-former is selected from the group consisting of calcium carbonate, calcium phosphate, calcium saccharide, calcium succinate, calcium tartrate, ferric acetate, ferric hydroxide, ferric phosphate, magnesium carbonate, magnesium citrate, magnesium hydroxide, magnesium phosphate, and mixtures thereof,
and/or wherein said dosage form comprises one or more active pharmaceutical ingredients selected from the group consisting of histamine H₂ receptor antagonists, proton pump inhibitors, 5-HT₃ agonists, histamine H₁ receptor antagonists, antiepileptic drugs, centrally acting adrenergic agonists, sleep aids, drugs for the treatment of erectile dysfunction, salts thereof and mixtures thereof,
and/or wherein said dosage form comprises one or more active pharmaceutical ingredients in sufficient quantities to be administered orally to a patient at prescribed dosing regimen to provide therapeutic efficacy.

14. A method of preparing a taste-masked multi-particulate composition in accordance with claim 11 wherein drug-containing beads are prepared by layering a pharmaceutically acceptable drug from a polymeric binder solution onto sugar spheres or cellulose spheres in fluid-bed equipment.

15. A method of preparing a taste-masked multi-particulate composition in accordance with claim 11 wherein said microgranules of one or more active pharmaceutical ingredient(s) are prepared by a high shear or planetary granulation process or said pellets are prepared by a granulation-extrusion-spheronization process comprising an active pharmaceutical ingredient, a polymer binder and one or more fillers/diluents.
